# EUROPÄISCHE PATENTSCHRIFT

(11) **EP 1 283 688 B1**
(45) Veröffentlichungstag und Bekanntmachung des Hinweises auf die Patenterteilung: **06.05.2004**
(21) Anmeldenummer: 01977951.1
(22) Anmeldetag: 05.02.2001
(51) Int. Cl.: A61B 3/135

(54) **VERFAHREN UND VORRICHTUNG ZUR ABBILDUNG EINES HINTEREN AUGENABSCHNITTES**
METHOD AND DEVICE FOR IMAGING A SECTION OF THE EYEGROUND
PROCEDE ET DISPOSITIF DE REPRESENTATION D'UNE COUPE DU FOND DE L'OEIL

(30) Priorität: 17.05.2000 CH 989002000
(43) Veröffentlichungstag der Anmeldung: 19.02.2003
(73) Patentinhaber: HAAG-STREIT AG, CH-3098 Köniz (CH)
(72) Erfinder: BARKER, Felix, Elkins Park, PA 19027-1598 (US)
(74) Vertreter: Roshardt, Werner Alfred, Dipl.-Phys.
(86) Internationale Anmeldenummer: PCT/CH2001/000076
(87) Internationale Veröffentlichungsnummer: WO 2001/087146

(56) Entgegenhaltungen:
- DE-B- 1 133 911

## Beschreibung

### Technisches Gebiet

Die Erfindung betrifft ein Verfahren und eine Vorrichtung, ein sogenanntes Spaltlampengerät, zur Abbildung eines hinteren Augenabschnittes eines Patientenauges mit einer ein Mikroskop aufweisenden Betrachtungseinheit und einer optischen Einheit.

### Stand der Technik

Die Untersuchung und Abbildung der hinteren Augenabschnitte, beispielsweise des Augenfundus, erfolgt typischerweise mit einem Spaltlampengerät, wobei vor dem entsprechenden Auge zusätzlich eine sogenannte Vorsatzlinse als optische Einheit positioniert wird. Diese Vorsatzlinse ermöglicht je nach Brennweite die Betrachtung unterschiedlich grosser Bereiche des Augenhintergrundes.

Um ungewollte Bewegungen des Patienten während einer Untersuchung zu vermeiden, wird der Kopf des Patienten in einem Kopfhalter fixiert. An diesem Kopfhalter ist typischerweise auch die Vorsatzlinse befestigt. Um jedoch eine präzise Positionierung der Vorsatzlinse vor dem Auge zu ermöglichen, muss diese Verbindung beweglich sein.

Im Journal of the American Optometric Association, Volume 59, Number 7, 7/88 ist eine Halterung für eine derartige Vorsatzlinse beschrieben. Die Halterung besteht aus einem mit Gelenken verbundenen Gestänge, welches mit einem Ende am Kopfhalter befestigt wird und am anderen Ende die optische Einheit trägt. Die Vorsatzlinse muss von Hand in der gewünschten Lage vor dem Auge positioniert werden, wobei die Reibung der Gelenke genügend gross ist, dass die Vorsatzlinse nach dem Loslassen mehr oder weniger in der eingestellten Lage verbleibt.

Das Problem ist allerdings, dass sich die Vorsatzlinse selbst von einem erfahrenen Anwender von Hand nur sehr schwer genügend präzise vor dem Auge positionieren lässt. Für einen Laien ist dies praktisch unmöglich.

In der DE 1133911 ist eine weitere Möglichkeit beschrieben, wie eine Vorsatzlinse vor dem Auge positioniert werden kann. Am Kopfhalter ist zu diesem Zweck ein Glaswagen mit einer Schaftführung, einem vertikalen Loch, befestigt. Der Wagen ist seitlich, jedoch nicht vertikal verschiebbar. Eine hierzu passende Vorsatzlinse besitzt einen zylindrischen Schaft, welcher in die Schaftführung am Wagen eingeführt werden kann. Dadurch ist die Vorsatzlinse sowohl seitlich (durch Verschieben des Wagens) als auch vertikal (durch Verschieben des Schaftes in der Schaftführung) beweglich. Die Halterung für die Vorsatzlinse selber ist beweglich mit dem Schaft verbunden, sodass auch der Abstand der Vorsatzlinse vom Auge in gewünschter Weise eingestellt werden kann. Durch eine Schraube an der Schaftführung kann die Vorsatzlinse in dieser fixiert werden. Zur einfacheren Positionierung kann die Vorsatzlinse auch bewegungsmässig mit dem Spaltlampengerät gekoppelt werden.

Auch hier gilt, dass eine Positionierung von Hand nicht genügend präzise ist. Zudem ist der Abkopplungsvorgang kompliziert und aufwändig und der Augenhintergrund lässt sich nicht mit einem Lichtspalt eines Spaltlampengerätes abscannen.

Ein weiteres Problem besteht darin, dass bei bekannten Linsenhaltern die optische Einheit zwar beweglich, aber fest, d.h. nicht lösbar, mit dem Linsenhalter verbunden ist. Bei einem Linsenwechsel muss daher der ganze Linsenhalter ausgewechselt werden.

### Darstellung der Erfindung

Aufgabe der Erfindung ist es, ein Verfahren der eingangs erwähnten Art anzugeben, welches die beim Stand der Technik vorhandenen Probleme vermeidet und insbesondere eine präzise Positionierung der Vorsatzlinse sowie eine ungehinderte Abbildung des hinteren Augenabschnittes erlaubt. Zudem soll ein Linsenhalter angegeben werden, welcher ein einfaches Auswechseln der optischen Einheit erlaubt.

Die Lösung der Aufgabe ist verfahrensmässig durch die Merkmale des Anspruchs 1 und vorrichtungsmässig durch die Merkmale des Anspruchs 4 definiert. Das bevorzugte Verfahren zur Abbildung der hinteren Augenabschnitte eines Patientenauges unter Verwendung einer Betrachtungseinheit und einer optischen Einheit umfasst zumindest die folgenden Schritte:
a) Die optische Einheit wird bewegungsmässig an die ein Mikroskop aufweisende Betrachtungseinheit gekoppelt.
b) Die optische Einheit wird derart vor dem Patientenauge positioniert, dass sie sich zwischen der Betrachtungseinheit und dem Patientenauge in einer gegebenen Relativposition zum Patientenauge befindet.
c) Die optische Einheit wird relativ zum Patientenauge räumlich fixiert.
d) Die optische Einheit wird unter Beibehaltung der räumlichen Fixierung relativ zum Patientenauge von der Betrachtungseinheit abgekoppelt.
e) Durch Verfahren der Betrachtungseinheit wird der hintere Augenabschnitt des Patientenauges abgebildet, wobei die Betrachtungseinheit im Wesentlichen in einer Ebene senkrecht zur optischen Achse des Auges verfahren und der Augenhintergrund beispielsweise mit einem Lichtspalt abgescannt wird.

Die bewegungsmässige Kopplung der optischen Einheit mit dem Mikroskop der Betrachtungseinheit ermöglicht es, die optische Einheit unter Verwendung der Betrachtungseinheit absolut präzise vor dem zu untersuchenden Auge zu positionieren. Die Position der optischen Einheit ist mit der Betrachtungseinheit jederzeit kontrollier- und durch Verschiebung der Betrachtungseinheit auch korrigierbar. Die räumliche Fixierung und die Abkopplung der optischen Einheit von der Betrachtungseinheit erfolgen auf einfache und schnelle Weise, ohne dass die Gefahr einer ungewollten Verschiebung der optischen Einheit besteht. Zudem schränkt die erfindungsgemäss abgekoppelte Vorsatzlinse die Bewegungsfreiheit der Betrachtungseinheit nicht ein, wodurch eine ungehinderte Abbildung der hinteren Augenabschnitte mit der Betrachtungseinheit ermöglicht wird. Somit können alle gewünschten Untersuchungen am Patientenauge ohne Umbau des Spaltlampengeräts durchgeführt werden.

Um einen möglichst umfassenden Betrachtungsbereich zu erreichen, wird die optische Einheit vorzugsweise derart vor dem Patientenauge positioniert, dass ihr Fokus bzw. ihr Brennpunkt auf der Pupillenebene des Patientenauges zu liegen kommt. Dabei fallen die optischen Achsen von Auge und optischer Einheit im Allgemeinen zusammen.

Zur einfacheren und sicheren Handhabung erfolgt die räumliche Fixierung der optischen Einheit relativ zum Patientenauge bevorzugt im Wesentlichen gleichzeitig mit der Abkopplung der optischen Einheit von der Betrachtungseinheit. Die Fixierung und die Abkopplung soll dabei mit möglichst wenigen Handgriffen, mit Vorteil natürlich mit einem einzigen Handgriff, beispielsweise durch Drücken eines Knopfes oder durch Betätigen eines Hebels, erfolgen.

Bei der Abbildung der hinteren Augenabschnitte wird der Abbildungsbereich durch die relativ kleine Puppen-Öffnung stark eingeschränkt, weshalb zwischen der Betrachtungseinheit und dem Auge eine optische Einheit platziert wird, indem diese an die Betrachtungseinheit gekoppelt wird. Durch Entkoppeln der optischen Einheit nach deren Positionierung wird bei der Abbildung der hinteren Augenabschnitte ein stark vergrösserter Abbildungsbereich erreicht.

Die erfindungsgemässe Vorrichtung zur Abbildung der hinteren Augenabschnitte mit einer Betrachtungseinheit und einer optischen Einheit umfasst Mittel zur bewegungsmässigen Kopplung der optischen Einheit mit der Betrachtungseinheit, Mittel zur Positionierung der mit der Betrachtungseinheit gekoppelten optischen Einheit zwischen der Betrachtungseinheit und dem Patientenauge, Mittel zur räumlichen Fixierung der optischen Einheit relativ zum Patientenauge sowie Mittel zum Entkoppeln der räumlich fixierten optischen Einheit von der Betrachtungseinheit.

Zur Abbildung der hinteren Augenabschnitte wird als Betrachtungseinheit bevorzugt ein Mikroskop, z.B. ein sogenanntes Greenough-Stereo-Mikroskop verwendet, wie es auch bei der Untersuchung von Augen verwendet wird. Bei solchen Untersuchungen wird der Kopf des Patienten meist örtlich fixiert. Das Mikroskop kann dann in allen Richtungen mehr oder weniger frei beweglich beliebig vor dem zu untersuchenden Auge positioniert werden. Als optische Einheit werden mit Vorteil sogenannte Vorsatzlinsen verwendet, welche zumeist an einem Linsenhalter befestigt sind.

Bei einer bevorzugten Ausführungsform der Erfindung erfolgt die örtliche Fixierung des Kopfes des Patienten (und damit auch des Patientenauges) in einem Kopfhalter, welcher einen Glaswagen umfasst, der horizontal beweglich am Kopfhalter befestigt ist. Die horizontale Bewegung des Glaswagens umfasst im Wesentlichen eine seitliche Verschiebung, sodass der Glaswagen und damit die optische Einheit wahlweise vor dem linken oder dem rechten Auge des Patienten platziert werden kann.

Eine entsprechend ausgebildete Vorsatzlinse ist am oberen Ende eines Linsenhalters mit einem zylindrischen Schaft befestigt, wobei der Schaft derart dimensioniert ist, dass er in eine entsprechende Schaftführung am Glaswagen eingeführt werden kann. Die Schaftführung besteht z.B. aus einem vertikal angeordneten Loch am Glaswagen, in welches der Schaft der Vorsatzlinse von oben her einführbar ist.

Zur bewegungsmässigen Kopplung der optischen Einheit mit der Betrachtungseinheit verfügt die Vorrichtung beispielsweise über eine der Betrachtungseinheit örtlich fest zugeordnete Führungsplatte mit einer Führungsnut. D.h. die Führungsplatte folgt genau den Bewegungen der Betrachtungseinheit, wobei die Führungsnut horizontal, parallel zur optischen Achse der Betrachtungseinheit, d.h. parallel zur Betrachtungsrichtung auf den Kopf des Patienten zu verläuft.

Die Kopplung wird erreicht, indem der in die Schaftführung am Glaswagen eingeführte Schaft des Linsenhalters mit seinem unteren Ende in die Führungsnut eingesetzt wird. Die Führungsnut und das untere Ende des Schaftes sind dabei derart ausgebildet, dass der Schaft in der Führungsnut nicht um seine Längsachse verdrehbar ist.

Der Schaft des Linsenhalters kann nicht nur direkt, sondern auch indirekt über einen Adapter in die Führungsnut eingesetzt werden. Der Adapter ist hierfür derart ausgebildet, dass er selber in die Führungsnut und der Schaft in den Adapter eingesetzt werden kann.

Diese Kopplung von Linsenhalter und Betrachtungseinheit erlaubt es, die seitlichen und vertikalen Bewegungen der Betrachtungseinheit auf die optische Einheit zu übertragen, wobei der Abstand der optischen Einheit zum Auge unverändert bleibt. Bei Bewegungen der Betrachtungseinheit auf das Auge zu oder vom Auge weg verschiebt sich lediglich der Schaft in der Führungsnut der Führungsplatte. Die Länge des Schaftes ist auf das Mikroskop abgestimmt, sodass die optische Achse der optischen Einheit im wesentlichen mit der optischen Achse der Betrachtungseinheit, welche im Falle eines Mikroskops die Symmetrieachse der beiden optischen Achsen des linksseitigen und des rechtsseitigen Teilsystems ist, zusammenfällt.

Die optische Einheit ist, beispielsweise über ein gelenkiges Parallelogramm, beweglich mit dem Schaft verbunden, wodurch sich der Frontalabstand der optischen Einheit zum Auge in gewünschter Weise einstellen lässt.

Die optische Einheit lässt sich somit beliebig vor dem zu untersuchenden Auge positionieren.

Bei einer bevorzugten Ausführungsform der Erfindung sind die Führungsplatte, der Linsenhalter und der Glaswagen derart ausgebildet, dass die optische Einheit in der gewünschten Position vor dem Patientenauge räumlich fixierbar ist. Der Linsenhalter kann in der Schaftführung am Glaswagen und/oder der Glaswagen am Kopfhalter fixiert werden. Die Fixierung erfolgt z.B. mechanisch oder magnetisch.

Eine bevorzugte Variante zur räumlichen Fixierung der optischen Einheit relativ zum Patientenauge erfolgt mechanisch über eine an der Schaftführung des Glaswagens angebrachte Hülse. Der Schaft des Linsenhalters wird durch die Hülse in die Schaftführung eingeführt und z.B. durch Drehen der Hülse in an sich bekannter Weise, beispielsweise durch Verengung des Innendurchmessers, in der Schaftführung festgeklemmt.

Weiter kann die Hülse beispielsweise einen Aussenzahnkranz aufweisen, welcher beim Drehen der Hülse einen Zahnradmechanismus in Gang setzt, der eine am Glaswagen angebrachte Bremse aktiviert.

Selbstverständlich kann der Linsenhalter auf beliebige Art und Weise fixiert werden. Eine weitere Möglichkeit besteht z.B. darin, den Schaft des Linsenhalters mit einem Knickgelenk und einem Spreizmechanismus auszurüsten, welcher durch Abknicken des unteren Endes ausgelöst wird. Gleichzeitig könnte mittels eines Hebelmechanismus die Bremse am Führungswagen aktiviert werden.

Eine weitere bevorzugte Art der räumlichen Fixierung der optischen Einheit basiert auf magnetischen Kräften. Hierbei werden der Linsenhalter und/oder der Glaswagen mittels eines oder mehrerer Magnete in der Schaftführung bzw. am Kopfhalter festgeklemmt.

Damit die hinteren Augenabschnitte mit dem Spaltlampengerät ungehindert abgescannt werden können, wird die optische Einheit nach deren Positionierung vor dem Auge von der Betrachtungseinheit abgekoppelt.

Der Linsenhalter, die Führungsplatte und der allenfalls vorhandene Adapter sind hierfür derart ausgebildet, dass die räumlich fixierte optische Einheit vorzugsweise durch horizontales und/oder vertikales Ausfahren bzw. durch Ausschwenken des unteren Endes des Schaftes, des Adapters oder der Führungsplatte von der Betrachtungseinheit abgekoppelt werden kann.

Zur Abkopplung ist beispielsweise die Führungsplatte über einen Federmechanismus ähnlich dem Minenvorschub eines Kugelschreibers mit der Betrachtungseinheit verbunden. Der Federmechanismus erlaubt das Hinunterdrücken der Führungsplatte und gibt auf diese Weise durch vertikales Ausfahren den Schaft des Linsenhalters frei. Der Federmechanismus ist dabei bevorzugt derart ausgebildet, dass die Führungsplatte in dieser Position einrastet und durch nochmaliges Drücken wieder gelöst werden kann.

Eine andere Variante besteht darin, dass, falls vorhanden, der Adapter über den oben genannten Federmechanismus verfügt. Nach dem Hinunterdrücken kann jedoch der Adapter von Hand entfernt werden, weshalb der Adapter nach dem Zusammendrücken nicht einrasten muss.

Bei einer weiteren Variante weist der Schaft der optischen Einheit beispielsweise ein Gelenk, insbesondere ein Knickgelenk (wie bereits weiter oben erwähnt) auf. Das Entkoppeln der optischen Einheit von der Betrachtungseinheit erfolgt durch Ausschwenken des unteren Endes des Schaftes aus der Führungsnut, z.B. durch eine manuelle Beugung des Gelenkes, wobei wie bereits beschrieben gleichzeitig der Linsenhalter räumlich fixiert werden kann.

Eine weitere Möglichkeit zur Entkopplung des Schaftes von der Führungsplatte besteht beispielsweise darin, den unteren Teil des Schaftes teleskopartig ausziehbar auszubilden. D.h. der Schaft besteht aus mindestens zwei zylindrischen Teilen, welche ineinandergeschoben werden können. Das Ineinanderschieben, d.h. das vertikale Herausheben des unteren Schaftendes aus der Führungsnut, könnte beispielsweise magnetisch durch Aktivierung eines entsprechenden Magneten erfolgen.

Selbstverständlich sind beliebige Kombinationen von mechanischer und magnetischer Fixierung bzw. Entkopplung der optischen Einheit möglich.

Die Positionierung der optischen Einheit vor dem Auge kann sowohl manuell als auch automatisch erfolgen, wobei selbstverständlich auch Mischformen möglich sind. Der Betrachter kann beispielsweise mit Hilfe der Betrachtungseinheit eine Grobpositionierung der optischen Einheit vornehmen und die Feinjustierung vor oder nach der Entkopplung der optischen Einheit von der Betrachtungseinheit durch eine Positionierautomatik durchführen lassen. Eine solche Positionierautomatik umfasst beispielsweise einen Pupillensensor zur Ermittlung der genauen Position der Pupille des zu untersuchenden Auges sowie eine Vorrichtung zur präzisen Positionierung der optischen Einheit relativ zur Pupille. Natürlich kann neben der Positionierung auch die Entkopplung und Fixierung der optischen Einheit nicht durch den Benutzer, sondern durch eine entsprechende Automatik erfolgen.

Bei einer besonders bevorzugten Ausführungsform der Erfindung erfolgt die Entkopplung der Linsenanordung von der Betrachtungseinheit im wesentlichen gleichzeitig mit der räumlichen Fixierung der optischen Einheit relativ zum Patientenauge, wobei beides ohne eine örtliche Verschiebung der optischen Einheit geschieht. Dabei bedeutet "im wesentlichen gleichzeitig", dass die Entkopplung und Fixierung mit einer bevorzugt einzigen Bewegung des Betrachters, z.B. durch Drücken eines Knopfes oder Betätigen eines Hebels erfolgen kann, wobei der Knopf oder der Hebel an einem geeigneten Ort, z.B. an der Betrachtungseinheit, am Kopfhalter oder an der optischen Einheit angebracht sein kann.

Zur Erstellung von Bildern der zu untersuchenden Augenabschnitte ist die Betrachtungseinheit vorzugsweise derart ausgebildet, dass eine Bilderfassungsvorrichtung mit dieser zusammenarbeitet. Über einen entsprechenden Kameraadapter kann beispielsweise eine Video- oder eine Fotokamera angeschlossen werden. Sehr gut geeignet sind auch Digitalkameras (z.B. CCD, CMOS), welche den Vorteil haben, dass die erfassten Bilder direkt digital weiterverarbeitet werden können.

Durch entsprechende Ausbildung des Kameraadapters oder der Kamera können auch weitere Funktionen wie beispielsweise ein Zoom oder eine Feinjustierung integriert werden. Dadurch wird es möglich, einen interessanten Bildausschnitt auszuwählen und vergrössert darzustellen.

Um ein einfaches und schnelles Wechseln, d.h. Austauschen der optischen Einheit zu ermöglichen, ist der Linsenhalter derart ausgebildet, dass die optische Einheit von Hand auf dem Linsenhalter festklemm- und wieder abnehmbar ist.

Eine bevorzugte Ausführungsform des Linsenhalters umfasst zwei federnd miteinander verbundene Halteteile, welche in einem bestimmten, der Dicke der optischen Einheit entsprechenden, Abstand voneinander angeordnet sind. Die optische Einheit kann auf diese Weise bequem und einfach zwischen diesen beiden Halteteilen eingeklemmt, und ebenso einfach wieder entfernt werden.

Der Einsatz eines solchen Linsenhalters beschränkt sich jedoch nicht auf die Anwendung zusammen mit der oben beschriebenen Vorrichtung. Er ist selbstverständlich überall dort einsetzbar, wo optische Einheiten, beispielsweise einzelne Linsen, in einen beliebigen Strahlengang eingebracht werden müssen.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: Eine erfindungsgemässe Vorrichtung zur Abbildung des Augenhintergrundes;
- Fig. 2: einen auf die Führungsplatte aufgesetzten Linsenhalter mit Linse;
- Fig. 3: einen Querschnitt durch die Führungsplatte mit Linsenhalter;
- Fig. 4: den Querschnitt aus Figur 3 bei zusammengedrückter Führungsplatte
- Fig. 5: eine seitliche Detailansicht des oberen Endes des Linsenhalters;
- Fig. 6: eine Frontansicht des oberen Endes des Linsenhalters;
- Fig. 7: einen Glaswagen mit Linsenhalter-Führung;
- Fig. 8: eine Detailansicht des Glaswagens von oben;
- Fig. 9: einen Adapter zur Kopplung des Linsenhalters mit dem Spaltlampengerät;
- Fig. 10: eine seitliche Detailansicht des unteren Endes des Linsenhalters mit einem Knickgelenk;
- Fig. 11: eine Detailansicht eines elektromagnetisch funktionierenden, teleskopartig ausgebildeten, unteren Endes des Linsenhalters bei ausgeschaltetem Strom;
- Fig. 12: den Linsenhalter aus Fig. 11 bei eingeschaltetem Strom und
- Fig. 13: mehrere Strahlengänge zur Fundus-Abbildung.

Grundsätzlich sind in den Figuren gleiche Teile mit gleichen Bezugszeichen versehen.

### Wege zur Ausführung der Erfindung

Im folgenden soll die Erfindung anhand einiger Beispiele im Detail erläutert werden.

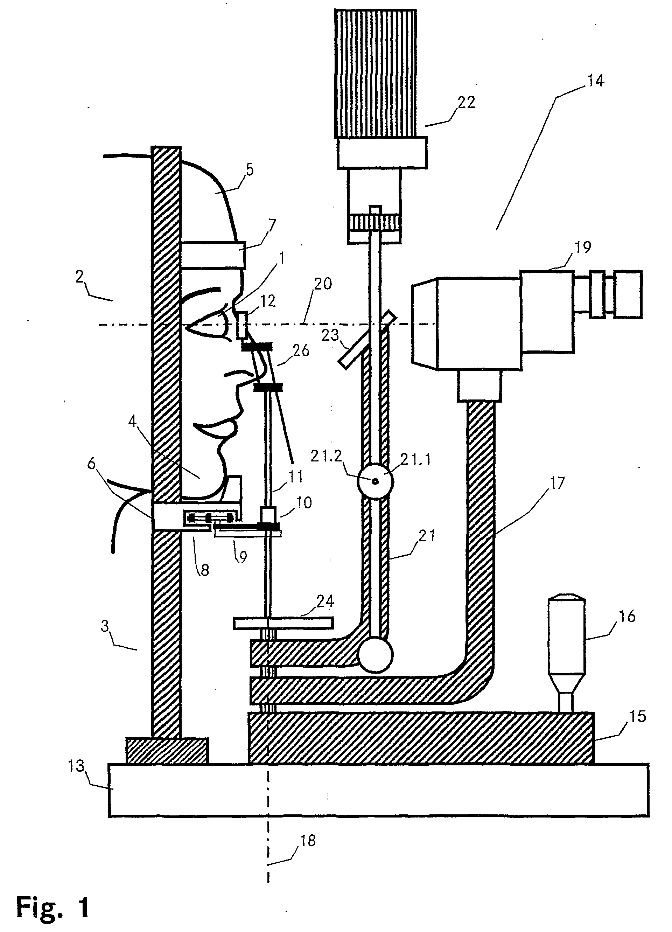

Figur 1 zeigt von der Seite ein auf einem Tisch 13 montiertes, erfindungsgemässes Spaltlampengerät 14 zur Abbildung der hinteren Augenabschnitte, insbesondere des Fundus (Augenhintergrund), des Auges 1 eines Patienten 2. Der Kopf des Patienten 2 ist in einem Kopfhalter 3 fixiert. Hierzu liegen Kinn 4 und Stirn 5 des Patienten an einer Kinnstütze 6 bzw. einer Stirnstütze 7 auf.

Die Kinnstütze 6 weist eine senkrecht zur Bildebene liegende Schiene 8 auf, in welcher ein Glaswagen 9 (senkrecht zur Bildebene) hin- und herbewegt werden kann. Der Glaswagen 9 umfasst eine Schaftführung 10, in welche ein Linsenhalter 11 eingeführt ist, welcher an seinem oberen Ende eine Linse 12 als sogenannte Vorsatzlinse trägt.

Auf der dem Patienten 2 gegenüberliegenden Seite des Kopfhalters 3 ist ein Spaltlampengerät 14 montiert. Dieses umfasst einen Kreuzschlitten 15 mit einem Lenkhebel 16, mit dessen Hilfe das Spaltlampengerät 14 horizontal (z.B. durch Neigen des Lenkhebels 16) sowie vertikal (z.B. durch Drehen des Lenkhebels 16) bewegt werden kann.

Die Linse 12 auf dem Linsenhalter 11 kann im Zusammenhang mit dem Spaltlampengerät 14 durch seitliches Verschieben des Glaswagens 9 sowie durch vertikales Verschieben in der Schaftführung 10 frei bewegt und natürlich auch vor dem Auge 1 positioniert werden.

Weiter ist auf einem Mikroskoparm 17, welcher um eine senkrecht stehende Drehachse 18 geschwenkt werden kann, ein Mikroskop 19, beispielsweise ein Greenough-Stereo-Mikroskop mit einer optischen Achse 20 befestigt (mit der optischen Achse 20 ist hier die Mittelsenkrechte der optischen Achsen des linksseitigen und des rechtsseitigen optischen Teilsystems des Mikroskops 19 gemeint).

An einem ebenfalls um die Drehachse 18 schwenkbaren Lampenarm 21 ist als Beleuchtungseinheit eine Spaltlampe 22 befestigt, deren Licht über einen Spiegel 23 in das zu untersuchende Auge 1 geworfen wird. Um störende Lichtreflexe im Auge 1 zu vermindern bzw. zu eliminieren ist die Spaltlampe 22 derart am Lampenarm 21 befestigt, dass sie um das Gelenk 21.1 mit senkrecht zur Bildebene stehender Drehachse 21.2 verschwenkt werden kann. Die Spaltlampe 22 erzeugt beispielsweise einen Lichtspalt, mit welchem der Augenhintergrund ausgeleuchtet wird. Durch seitliches und/oder vertikales Verschieben des Spaltlampengeräts kann der gesamte Abbildungsbereich abgescannt werden. Zusätzlich zur Spaltlampe 22 kann, beispielsweise an einem beweglichen Arm, eine weitere Lichtquelle, z.B. der Ausgang eines Lichtleiters, vorgesehen sein, welche beispielsweise zur Erhellung des gesamten Augenhintergrundes dient, während mit dem von der Spaltlampe 22 erzeugten Lichtspalt nur ein schmaler Streifen des Fundus stark erhellt wird.

Weiter umfasst die Vorrichtung eine ebenfalls um die Drehachse 18 drehbare Führungsplatte 24, welche mit dem Mikroskoparm 17 gekoppelt ist, sodass sie dessen Drehbewegungen mitmacht.

Auf der Oberseite der Führungsplatte 24 befindet sich eine (in der Figur 1 nicht sichtbare) Führungsnut 24.1. Bedingt durch die Drehkopplung der Führungsplatte 24 mit dem Mikroskoparm 17 verläuft diese Führungsnut 24.1 stets parallel zur optischen Achse 20 des Mikroskops 19.

In diese Führungsnut 24.1 ist das untere Ende des in die Schaftführung 10 eingeführten Linsenhalters 11 eingesetzt.

Auf diese Weise ist die Linse 12 am oberen Ende des Linsenhalters 11 bewegungsmässig mit dem Mikroskop 19 gekoppelt. Bei seitlichen (horizontal, senkrecht zur optischen Achse 20) und vertikalen Bewegungen des Mikroskops 19 wird die Linse 12 via Führungsplatte 24 und Linsenhalter 11 mitbewegt. Bei Bewegungen in Richtung der optischen Achse 20 vor oder zurück gleitet der Linsenhalter 11 in der Führungsnut 24.1 vor und zurück, ohne dass sich der Abstand der Linse 12 zum Auge 1 verändert. Um diesen Abstand einstellen zu können, ist die Linse 12 beispielsweise über ein Parallelgelenk 26 mit dem Linsenhalter verbunden.

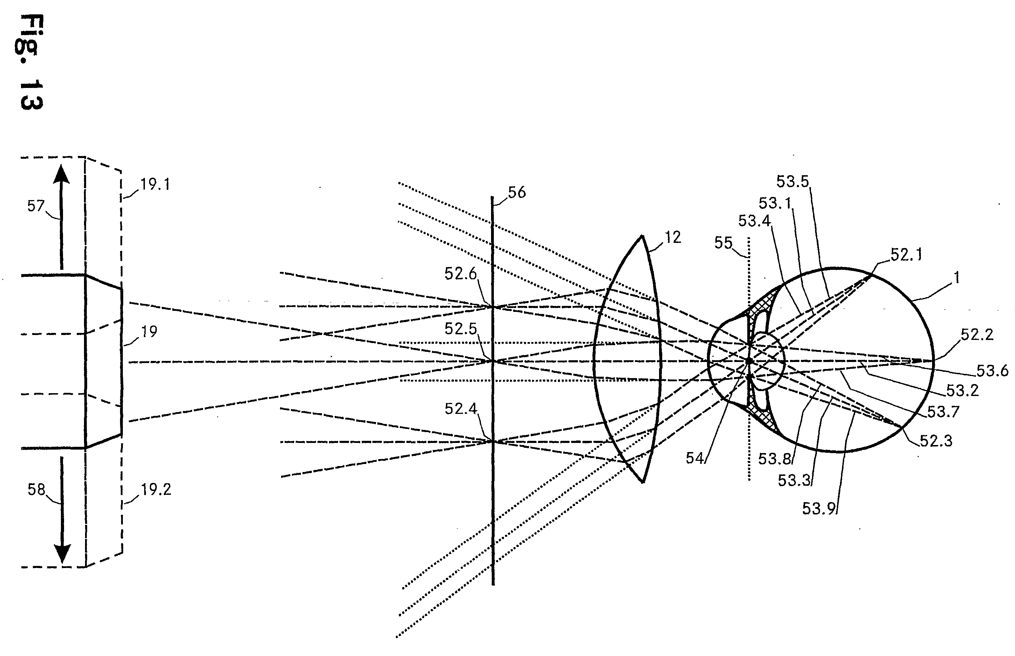

Bei der Abbildung des Augenhintergrundes liegt somit die Linse 12 genau in der optischen Achse 20 zwischen dem Auge 1 und dem Mikroskop 19. Wie bereits erwähnt, ermöglicht es die Linse 12, mit dem Mikroskop 19 einen grossen Bereich des Augenfundus zu betrachten, was die Figur 13 veranschaulicht. Der Einfachheit halber ist in der Figur 13 nur der Strahlengang für drei Funduspunkte 52.1, 52.2, 52.3 des Auges 1 dargestellt. Für jeden Funduspunkt 52.1, 52.2, 52.3 sind jeweils der Hauptstrahl 53.1, 53.2 und 53.3 sowie die beiden Randstrahlen 53.4, 53.5, 53.6, 53.7, 53.8 und 53.9, welche gerade noch durch die Pupille des Auges 1 nach aussen gelangen, dargestellt. Die Hauptstrahlen 53.1 - 53.3 sowie die Randstrahlen 53.4 - 53.9 werden an der Hornhaut, d.h. der Grenzschicht zwischen Auge 1 und Luft gebrochen. Sämtliche von einem einzigen Punkt des Augenfundus stammenden Strahlen verlaufen (ein gesundes Auge 1 vorausgesetzt) ausserhalb des Auges 1 parallel. Die Brechung der Strahlen an der Augenlinse ist gegenüber der Brechung an der Hornhaut vernachlässigbar und deshalb nicht dargestellt, Wäre die Linse 12 nicht vorhanden, würden diese Strahlen 53.1 - 53.9) geradlinig weiter verlaufen (als gepunktete Linien dargestellt).

Durch die Linse 12 werden sämtliche Strahlen eines einzigen Funduspunktes 52.1 - 52.3 auf eine Bildebene 56 abgebildet, sodass ein Bild 52.4 - 52.6 entsteht. Der Abstand zwischen der Linse 12 und der Pupillenebene 55 korrespondiert in etwa mit der fokalen Länge der Linse 12. Auf diese Weise ist die telezentrische Bedingung erfüllt und die Hauptstrahlen 53.1, 53.2, 53.3 sind alle parallel zur optischen Achse 20 in der Richtung des Mikroskops 19.

Um dieses Bild in der Bildebene 56 mit dem Mikroskop 19 betrachten zu können, muss das Mikroskop 19 nur noch auf die Bildebene 56 fokussiert werden. Um sämtliche Bereiche des Fundus betrachten bzw. abscannen zu können, wird das Mikroskop 19 in einer zur Bildebene 56 parallelen Ebene verschoben, ohne den Abstand des Mikroskops 19 zur Bildebene 56 bzw. zum Auge 1 zu verändern. Diese Verschiebung ist durch die beiden Pfeile 57 und 58 dargestellt. Gestrichelt eingezeichnet sind zwei verschiedene Positionen des verschobenen Mikroskops 19.1 und 19.2. Diese einfache Methode der Einstellung ist nur möglich, wenn die Distanz zwischen der Linse 12 und der Pupillenebene 55 so gewählt ist, dass die telezentrische Bedingung erfüllt ist.

Weiter erlaubt es diese Verschiebung, von dem zu betrachtenden Fundusbereich einen möglichst grossen Lichtstrom aufzufangen, d.h. ein möglichst helles Bild des betrachteten Fundusbereichs zu erhalten.

Um dem Betrachter das zur Bildebene 56 parallele Verschieben des Mikroskops 19 zu erleichtern, könnte der Kreuzschlitten 15 beispielsweise mit einer Art Bremse ausgestattet werden, welche nach der Positionierung der Linse 12 und des Mikroskops 19 zugeschaltet werden könnte. Diese Bremse würde dann für einen erhöhten Bewegungswiderstand des Kreuzschlittens 15 in Richtung auf das Auge 1 zu (bzw. von diesem weg) sorgen oder solche Mikroskopverschiebungen vollständig verunmöglichen. Eine Blockierung des Kreuzschlittens 15 ist allerdings nicht sehr vorteilhaft, denn auch bei noch so genauer Positionierung sind manchmal kleine Korrekturen des Mikroskopabstandes notwendig.

Vorzugsweise werden positive Linsen 12 verwendet, die ein reelles Bild des Augenfundus zwischen der Linse 12 und dem Mikroskop erzeugen, wobei aber auch negative Linsen 12 verwendet werden können, die (vom Mikroskop aus gesehen hinter der Linse 12) ein virtuelles Bild erzeugen.

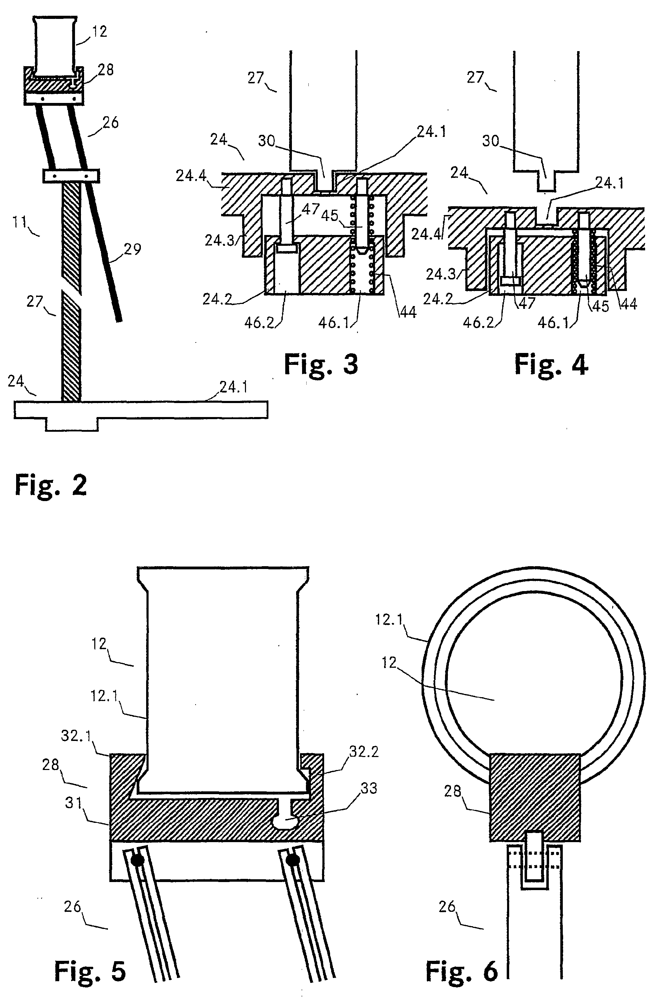

Figur 2 zeigt den Linsenhalter 11 und die Führungsplatte 24 in einem grösseren Massstab. Der Linsenhalter 11 umfasst einen Schaft 27 und an dessen oberen Ende das Parallelgelenk 26. Dieses besteht aus einem parallelogrammartig ausgebildeten Gestänge, dessen Seiten gelenkig miteinander verbunden sind. Eine Seite des Parallelogramms steht senkrecht zum Schaft 27 und ist mit dessen oberem Ende fest verbunden. Mit der zu dieser Seite parallelen Seite ist eine Klemmvorrichtung 28 verbunden, in welche die Linse 12 auf einfache Art und Weise von Hand auf dem Linsenhalter 11 festgeklemmt sowie wieder entfernt werden kann. Dies ist besonders hilfreich, wenn die Linse 12 während der Untersuchung durch eine andere Linse ausgetauscht werden soll. Zur Veränderung des Abstandes der Linse 12 zum Auge 1 ist eine der anderen beiden Seiten verlängert und bildet einen Verstellhebel 29.

In den Figuren 3 und 4 ist ein Längsschnitt durch das untere Ende des Schaftes 27 und der Führunsplatte 24 dargestellt. Am unteren Ende des Schaftes 27 ist eine Passfeder 30 ausgebildet, welche in einer Führungsnut 24.1 der Führungsplatte 24 steckt. Die Passfeder 30 erstreckt sich über die ganze Breite des Schaftes 27 und liegt parallel zur optischen Achse einer auf dem Linsenhalter 11 montierten Linse 12.

Die Führungsplatte 24 besteht aus einem Unterteil 24.2 und einem Oberteil, das aus einer Platte 24.4 mit der Führungsnut 24.1 und einer Art Hohlzylinder 24.3 besteht, der auf der Unterseite der Platte 24.4 angebracht ist. Der Aussendurchmesser des Unterteils 24.2 ist kleiner als der Innendurchmesser des Hohlzylinders 24.3. Beide sind derart ausgebildet, dass das Unterteil 24.2 von unten her in den Hohlzylinder 24.3 geschoben werden kann.

Eine Schraubenfeder 44 ist derart zwischen dem Ober- und dem Unterteil 24.2 befestigt, dass sie die ineinandergeschobenen Teile auseinanderdrückt. Damit die beiden Teile beim ineinander- bzw. auseinanderschieben nicht verkanten, ist innen im Hohlzylinder 24.3 ein Führungsstift 45 montiert, der in einer Führungsbohrung 46.1 am Unterteil 24.2 geführt wird. Die Führungsbohrung 46.1 wird gleichzeitig zur Führung der Schraubenfeder 44 benutzt. Damit das Ober- bzw. das Unterteil 24.2 durch die Schraubenfeder 44 nicht zu weit auseinandergedrückt werden, ist eine weitere Führungsbohrung 46.2 im Unterteil 24.2 vorgesehen, durch welche eine Schraube 47 von unten in die Platte 24.4 des Oberteils geschraubt wird. Der Kopf der Schraube 47 dient als Anschlag, damit der Hohlzylinder 24.3 und das Unterteil 24.2 nicht auseinanderfallen. Die Schraube dient gleichzeitig auch zur Verbesserung der Führung des Unterteils 25.2 im Hohlzylinder 24.3.

Das Oberteil weist auf seiner Oberseite eine Führungsnut 24.1 auf, in welche die Passfeder 30 des Schaftes 27 gesteckt werden kann. Die Führungsnut 24.1 erstreckt sich durchgehend über die gesamte Länge der Führungsplatte 24, sodass der in die Führungsnut 24.1 eingesteckte Schaft 27 horizontal in Richtung der Führunsnut 24.1 bewegbar ist. Die Führungsplatte 24 selber ist wie bereits erwähnt um die Drehachse 18 drehbar mit dem Mikroskoparm 17 verbunden, sodass sie eine Drehbewegung des Mikroskoparms 17 mitmacht.

Figur 3 zeigt den Querschnitt durch die Führungsplatte 24 im auseinandergedrückten Zustand, d.h. bei (mehr oder weniger) entspannter Schraubenfeder 44 und eingestecktem Schaft 27 des Linsenhalters 11. Figur 4 hingegen zeigt die Führungsplatte 24 im zusammengedrückten Zustand. Die Schraubenfeder 44 ist komprimiert und der (in der Führung 10 am Glaswagen 9 fixierte) Schaft 27 steckt nicht mehr in der Führungsnut 24.1. Durch das Zusammendrücken wird der Schaft 27 freigegeben und der Linsenhalter 11 bzw. die Linse 12 damit bewegungsmässigvon der Führungsplatte 24 bzw. vom Mikroskop 19 entkoppelt.

Durch einen nicht dargestellten Fixierungsmechanismus (ähnlich wie bei der Fixierung einer Kugelschreibermine in Schreib- bzw. Transportposition) rastet die Führungsplatte beispielsweise in der nach unten gedrückten Position ein und gibt die Passfeder 30 des Linsenhalters 11 frei. Danach können die gewünschten Untersuchungen durchgeführt werden, wobei die Führungsplatte 24 zur Vergrösserung der Bewegungsfreiheit des Spaltlampengeräts zuvor auch noch vollständig entfernt werden kann. Durch Wiedereinsetzen und nochmaliges Drücken auf die Führungsplatte 24 wird die Arretierung beispielsweise gelöst und die Führungsplatte befindet sich wieder in der ursprünglichen Position.

Die Figuren 5 und 6 zeigen einen Querschnitt bzw. eine Frontalansicht der Klemmvorrichtung 28 mit aufgestecker Linse 12, welche in einer Linsenfassung 12.1 eingefasst und am Parallelgelenk 26 befestigt ist. Die Klemmvorrichtung 28 umfasst eine Basis 31 sowie ein vorderes und ein hinteres Halteteil 32.1 und 32.2, welche auf der Basis 31 in einem bestimmten Abstand zueinander befestigt sind. Der Abstand der beiden Halteteile 32.1 und 32.2 an der Basis 31 ist entweder fest auf die Dicke einer bestimmten Linse bzw. auf die Breite der Linsenfassung abgestimmt oder er ist variabel einstellbar. Zudem sind die beiden Halteteile 32.1 und 32.2 derart ausgebildet, dass ihr gegenseitiger Abstand kleiner wird mit zunehmendem Höhenabstand von der Basis 31.

Die Basis 31 und/oder die Halteteile 32.1 und 32.2 bestehen aus einem elastischen Material, damit eine Linse 12, deren Linsenfassung 12.1 breiter als der kleinste Abstand der beiden Halteteile 32.1 und 32.2 ist, zwischen diesen eingeklemmt werden kann.

Um die Federwirkung der Halteteile 32.1 und 32.2 zu vergrössern, ist in der Basis 31 ein Schlitz 33 ausgebildet.

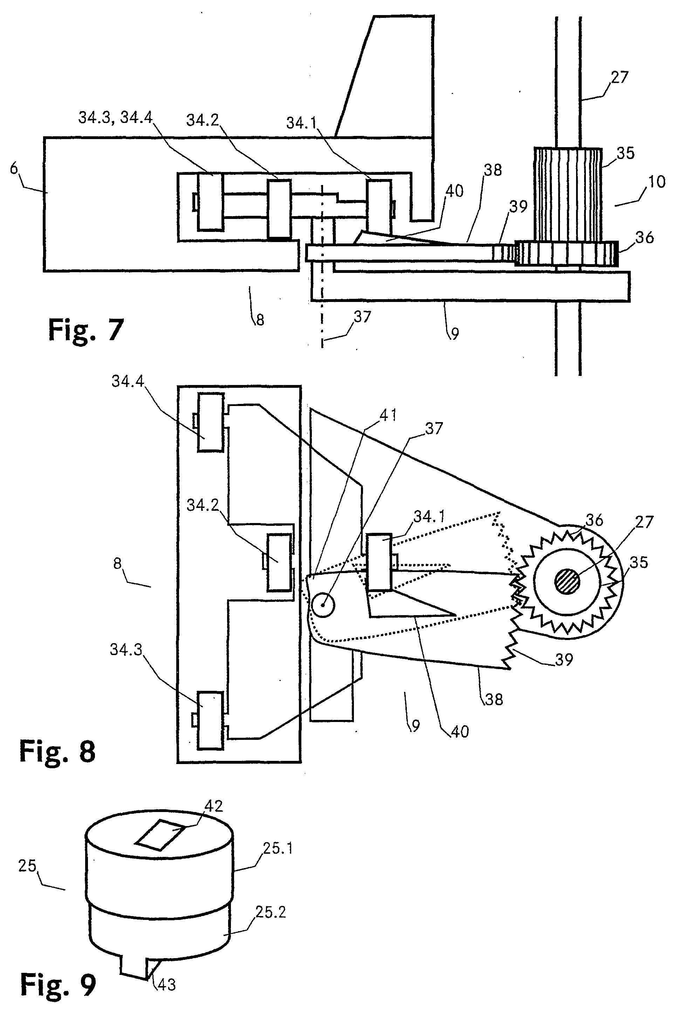

In Figur 7 ist aus seitlicher Sicht die Kinnstütze 6 mit dem in der Schiene 8 laufenden Glaswagen 9 dargestellt. Der Glaswagen 9 läuft auf mehreren Rollen 34.1, 34.2, 34.3 bzw. 34.4. Die Schaftführung 10 am Glaswagen 9 umfasst eine Rändelschraube 35 (die auch nur als Hülse ausgeführt sein kann), welche an ihrem unteren Ende einen Zahnkranz 36 aufweist. Durch Drehen der Rändelschraube 35 wird einerseits der Schaft 27 eines in die Schaftführung 10 eingeführten Linsenhalters 11 in der Schaftführung 10 festgeklemmt. Dies erfolgt z.B. in bekannter Art und Weise mittels Verkleinerung des Innendurchmessers der Schaftführung 10.

Weiter ist am Glaswagen 9 ein um eine Drehachse 37 drehbar gelagerter Exzenter 38 vorgesehen, welcher über einen weiteren, nicht vollständig ausgebildeten Zahnkranz 39 verfügt, dessen Zähne in den Zahnkranz 36 der Schaftführung 10 eingreifen. Durch Drehen der Rändelschraube 35 wird somit nicht nur der Schaft 27 festgeklemmt, sondern zusätzlich auch der Exzenter 28 um die Drehachse 37 verdreht. Auf dem Exzenter 38 befindet sich eine Erhöhung 40, welche durch die Drehung des Exzenters 38 unter die Rolle 34.1 zu liegen kommt und diese blockiert, indem sie beispielsweise an die Schiene 8 gepresst wird. Dadurch wird die Bewegung des Glaswagens 9 in der Schiene 8 gebremst oder vollständig blockiert.

Eine weitere Möglichkeit, den Glaswagen 9 in der Schiene zu blockieren besteht darin, dass eine Aussenkante des Exzenters 38 derart ausgebildet ist, dass sie durch dessen Drehung an die Schiene 8 gepresst wird.

In der Figur 8, welche von oben gesehen den Glaswagen 9 (zumindest teilweise) mit der Schaftführung 10 und einen Teil der Schiene 8 zeigt, ist beispielsweise eine spezielle Form mit einer Ausbuchtung 41 des Exzenters 38 dargestellt. In der nicht blockierenden Position (ausgezogene Linien) befindet sich die Ausbuchtung 41 in einem bestimmten Abstand zur Schiene 8, sodass sich der Glaswagen 9 beliebig seitlich verschieben lässt. Wird die Rändelschraube 35 im Uhrzeigersinn gedreht, nähert sich die Ausbuchtung 41 der Schiene 8, bis sie diese schliesslich berührt und dagegen gepresst wird (gepunktete Linien). Dadurch ist der Glaswagen blockiert.

Anstatt die Führungsplatte 24 derart auszubilden, dass sie durch Zusammendrücken aus dem Schaft 27 ausgefahren werden kann, kann auch ein Adapter 25 verwendet werden, wie ihn die Figur 9 in einer perspektivischen Darstellung zeigt. Der Adapter besteht aus einem Oberteil 25.1, eine Art Hohlzylinder mit Deckel, und einem Unterteil 25.2, dessen Aussendurchmesser kleiner als der Innendurchmesser des Oberteils 25.1 ist. Beide sind derart ausgebildet, dass das Unterteil 25.2 von unten her in das Oberteil 25.1 geschoben werden kann. Das Oberteil 25.1 entspricht dabei dem Hohlzylinder 24.3 und das Unterteil 25.2 dem Unterteil 24.2 der Führungsplatte 24 aus den Figuren 3 und 4. Das Oberteil 25.1 weist auf seiner Oberseite eine Nut 42 auf, in welche die Passfeder 30 des Schaftes 27 gesteckt werden kann. Die Nut 42 ist jedoch nicht durchgehend, sodass der in den Adapter 25 eingesteckte Schaft 27 horizontal völlig fixiert ist. Der Unterteil 25.2 des Adapters 25 weist auf seiner Unterseite eine in die Führungsnut 24.1 der Führungsplatte 24 passende Passfeder 43 auf.

Im Adapter 25 befindet sich dieselbe Mechanik, wie sie die Figuren 3 bzw. 4 für die Führungsplatte 24 zeigen. Diese Ausbildung des Adapters 25 erlaubt es, ihn mit seiner Passfeder 43 in die Führungsnut 24.1 der Führungsplatte 24 und die Passfeder 30 des Schaftes 27 eines in die Schaftführung 10 eingeführten Linsenhalters 11 in die Nut 42 des Adapters 25 einzusetzen. Auf diese Weise wird die am Linsenhalter 11 befestigte Linse 12 bewegungsmässig mit dem Mikroskop 19 gekoppelt.

Zur Fixierung des Linsenhalters 11 nach der Positionierung vor dem Auge 1 des Patienten 2 wird die Rändelschraube 35 im Uhrzeigersinn gedreht. Dadurch wird einerseits der Linsenhalter 11 in der Schaftführung 10 und via Exzenter 38 andererseits der Glaswagen 9 in der Schiene 8 der Kinnstütze 6 festgeklemmt.

Die Entkopplung des Linsenhalters 11 und damit der Linse 12 vom Mikroskop 19 unter Beibehaltung der räumlichen Fixierung erfolgt durch nach unten Drücken des Oberteils 25.1 des Adapters 25. Dadurch wird die Passfeder 30 des Linsenhalters 11, welche zuvor in der Nut 42 des Oberteils 25.1 eingesteckt war, freigegeben (siehe auch Fig. 4). Danach kann der Adapter 25 selber durch horizontale Verschiebung in der Führungsnut 24.1 aus der Führungsplatte 24 entfernt werden. (Eine Arretierung in zusammengedrückter Position ist in diesem Beispiel nicht unbedingt nötig.)

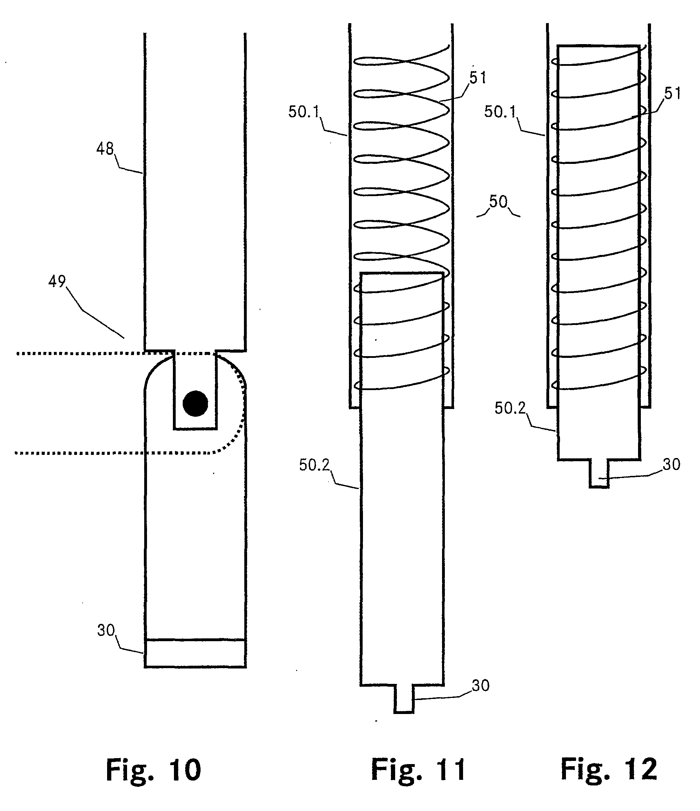

Figur 10 zeigt ein weiteres Beispiel für den Schaft 48 eines Linsenhalters 11. Das untere Ende des Schaftes 48 mit der Passfeder 30 ist über ein Knickgelenk 49 mit dem Rest des Schaftes 48 verbunden. Das Knickgelenk 49 ist so ausgebildet, dass es sowohl in gestreckter Position (ausgezogene Linien), als auch in umgeknickter Position (gepunktete Linien) arretiert werden kann. Das Umknicken des Schaftendes erfolgt in Längsrichtung der Passfeder 30, d.h. die Drehachse des Knickgelenkes steht senkrecht zur Bildebene. Dadurch lässt sich der räumlich fixierte Linsenhalter 11 durch einfaches Umknicken des unteren Schaftendes bewegungsmässig vom Mikroskop 19 abkoppeln.

Selbstverständlich ist es auch möglich, nicht nur die Abkopplung, sondern auch die räumliche Fixierung des Linsenhalters 11 durch Umknicken des Schaftendes zu realisieren. Beispielsweise könnte beim Umknicken des unteren Ende des Schaftes 48 über einen sich im Innern des Schaftes 48 befindenden Hebelmechanismus die Rändelschraube gedreht werden oder es wird direkt ein Mechanismus zur Fixierung des Linsenhalters 11 in der Schaftführung 10 sowie zur Fixierung des Glaswagens 9 in der Schiene 8 betätigt.

Die Figuren 11 und 12 zeigen ein weiteres Beispiel zur Ausgestaltung des Schaftes 50 des Linsenhalters 11. Der Schaft 50 ist zweiteilig ausgeführt. Er umfasst ein als Hohlzylinder ausgebildetes oberes Teil 50.1 und ein zylindrisch ausgebildetes unteres Teil 50.2, wobei der Aussendurchmesser des unteren Teils 50.2 kleiner als der Innendurchmesser des oberen Teils 50.1 ist. Das untere Teil 50.2 ist aus magnetisierbarem Material hergestellt und weist an seinem unteren Ende wiederum die Passfeder 30 auf. Auf der Innenseite des oberen Teils 50.1 ist ein elektrischer Leiter spiralförmig zu einer Spule 51 aufgewickelt, dessen Enden elektrisch leitend mit einer (nicht dargestellten) elektrischen Stromversorgung verbunden sind. Die Stromversorgung besteht z.B. aus einer Batterie im oberen Teil 50.1 des Schaftes 50. Die Zuführung des Stromes kann auch von einer externen Stromversorgung her, z.B. via Führungsplatte 24, via Glaswagen 9 oder via frei geführte Kabel erfolgen.

Wird, z.B. durch Drücken eines Knopfes, an den Enden des Leiters der Spule 51 eine konstante elektrische Spannung angelegt, wird im Innern der Spule 51 ein Magnetfeld generiert, das das untere Teil 50.2 des Schaftes 50 in die Spule 51 hinein und damit nach oben zieht.

Selbstverständlich kann auch eine Fixierung des unteren Teils 50.2 in angezogener und/oder in ausgefahrener Position vorgesehen sein. Oder das untere Teil 50.2 ist als Magnet, z.B. als Stabmagnet ausgebildet und wird durch Einschalten des Stromes nach oben gezogen oder nach unten gedrückt. Weiter können Zug- oder Druckfedern vorhanden sein, welche das untere Teil 50.2 nach der Auslenkung durch das Magnetfeld wieder in die ursprüngliche Position zurückbewegen.

Die Fixierung des Schaftes 50 in der Schaftführung 10 und/oder des Glaswagens 9 in der Schiene 8 kann auch hier auf verschiedene Weise erfolgen: entweder mechanisch in der bereits dargestellten Weise, indem durch das nach oben gezogene untere Teil 50.2. des Schaftes die Rändelschraube gedreht wird oder ebenfalls magnetisch, indem durch das erzeugte Magnetfeld beispielsweise ein beweglich montiertes Teil des Glaswagens 9 zum Linsenhalter 11 gezogen wird und auf diese Weise Linsenhalter und Glaswagen fixiert.

Eine weitere Art der magnetischen Fixierung erfolgt beispielsweise, indem durch das Umknicken des unteren Endes des Schaftes 48 aus Figur 10 ein Magnet in einen magnetischen Kreis eingebracht wird und diesen dadurch schliesst. Der magnetische Kreis verläuft z.B. vom Linsenhalter 11 via Schaftführung 10 und Glaswagen 9 zur Schiene 8 und wieder zurück zum Linsenhalter 11. Die Fixierung könnte beispielsweise erfolgen, indem ausgenutzt wird, dass die magnetischen Kräfte nach dem Schliessen des magnetischen Kreises versuchen, den magnetischen Widerstand zu reduzieren, indem bewegliche Teile des magnetischen Kreises minimal verschoben werden (d.h. dass die entsprechenden Luftspalten verkleinert werden) und auf diese Art den Linsenhalter und den Glaswagen festklemmen.

Zusammenfassend ist festzustellen, dass es die erfindungsgemässe Vorrichtung erlaubt, die hinteren Augenabschnitte auf optimale Art und Weise abzubilden, indem die zur Abbildung notwendige Vorsatzlinse mit Hilfe des Mikroskops sehr präzise vor dem zu untersuchenden Auge positioniert und danach auf einfache Art und Weise räumlich fixiert und im gleichen Arbeitsschritt vom Mikroskop abgekoppelt werden kann. Die Untersuchung erfolgt anschliessend mit dem frei beweglichen Mikroskop.

## Patentansprüche

1. Verfahren zur Abbildung eines hinteren Augenabschnittes eines Patientenauges (1) mit einer Betrachtungseinheit (19) und einer optischen Einheit (12), **dadurch gekennzeichnet, dass** es zumindest folgende Schritte umfasst:
a) die optische Einheit wird bewegungsmässig an die Betrachtungseinheit gekoppelt,
b) sie wird derart vor dem Patientenauge positioniert, dass sie sich zwischen der Betrachtungseinheit und dem Patientenauge in einer gegebenen Relativposition zum Patientenauge befindet,
c) die optische Einheit wird relativ zum Patientenauge räumlich fixiert,
d) die optische Einheit wird unter Beibehaltung der räumlichen Fixierung relativ zum Patientenauge von der Betrachtungseinheit abgekoppelt,
e) durch Verfahren der Betrachtungseinheit wird der hintere Augenabschnitt des Patientenauges betrachtet bzw. abgebildet.

2. Verfahren nach Anspruch 1, **dadurch gekennzeichnet, dass** die optische Einheit derart vor dem Patientenauge positioniert wird, dass ein Fokus der optischen Einheit auf eine Pupillenebene des Patientenauges zu liegen kommt.

3. Verfahren nach Anspruch 1 oder 2, **dadurch gekennzeichnet, dass** die räumliche Fixierung und die Abkopplung der Linsenanordung im wesentlichen gleichzeitig erfolgen.

4. Vorrichtung, insbesondere Spaltlampengerät, mit einer Betrachtungseinheit und einer optischen Einheit zur Abbildung eines hinteren Augenabschnittes eines Patientenauges, **gekennzeichnet durch** Mittel zur bewegungsmässigen Kopplung der optischen Einheit mit der Betrachtungseinheit, Mittel zur Positionierung der mit der Betrachtungseinheit gekoppelten optischen Einheit zwischen der Betrachtungseinheit und dem Patientenauge, Mittel zur räumlichen Fixierung der optischen Einheit relativ zum Patientenauge sowie Mittel zum Entkoppeln der räumlich fixierten optischen Einheit von der Betrachtungseinheit.

5. Vorrichtung nach Anspruch 4, **dadurch gekennzeichnet, dass** die Betrachtungseinheit ein Mikroskop (19), beispielsweise ein Greenough-Mikroskop, und die optische Einheit eine Vorsatzlinse (12) ist.

6. Vorrichtung nach Anspruch 4 oder 5, **dadurch gekennzeichnet, dass** sie einen Kopfhalter (3) zur örtlichen Fixierung eines Patienten-Kopfes und somit des Patientenauges aufweist und die Mittel zur Kopplung der optische Einheit mit der Betrachtungseinheit einen am Kopfhalter horizontal beweglich befestigten Glaswagen (9), einen Linsenhalter (11) mit einem Schaft (27) sowie eine der Betrachtungseinheit örtlich fest zugeordnete Führungsplatte (24) mit einer Führungsnut (24.1) aufweisen, wobei die optische Einheit am Linsenhalter befestigt ist und der Schaft in eine entsprechende Schaftführung (10) am Glaswagen einführbar sowie relativ zum fixierten Patientenauge positionierbar ist und die optische Einheit durch Einsetzen des unteren Endes des in die Schaftführung am Glaswagen eingeführten Schaftes in die Führungsnut bewegungsmässig mit der Betrachtungseinheit koppelbar ist, wobei der Schaft insbesondere verdrehsicher, direkt oder über einen Adapter (25) in die Führungsnut einsetzbar ist.

7. Vorrichtung nach Anspruch 6, **dadurch gekennzeichnet, dass** der Linsenhalter in der Schaftführung am Glaswagen und/oder der Glaswagen am Kopfhalter mechanisch oder bevorzugt magnetisch räumlich fixierbar ist.

8. Vorrichtung nach Anspruch 7, **dadurch gekennzeichnet, dass** die Schaftführung am Glaswagen eine Hülse (35) mit einem Aussenzahnkranz (36) aufweist, wobei durch Drehen der Hülse ein Klemmmechanismus zum Festklemmen des Schaftes in der Hülse betätigbar ist und/oder via Aussenzahnkranz eine Bremse zum Festklemmen des Glaswagens am Kopfhalter aktivierbar ist.

9. Vorrichtung nach einem der Ansprüche 6 bis 8, **dadurch gekennzeichnet, dass** der Linsenhalter, die Führungsplatte sowie der allenfalls vorhandene Adapter derart ausgebildet sind, dass die räumlich fixierte optische Einheit durch horizontales und/oder vertikales Ausfahren bzw. Ausschwenken des unteren Endes des Schaftes, der Führungsplatte oder des Adapters bewegungsmässig von der Betrachtungseinheit abkoppelbar ist.

10. Vorrichtung nach den Ansprüchen 4 bis 9, **dadurch gekennzeichnet, dass** sie derart ausgebildet ist, dass die optische Einheit im Wesentlichen gleichzeitig von der Betrachtungseinheit abkoppel- und ohne örtliche Verschiebung relativ zum Patientenauge räumlich fixierbar ist.

11. Vorrichtung nach einem der Ansprüche 4 bis 10, **dadurch gekennzeichnet, dass** eine Bilderfassungsvorrichtung, insbesondere eine Foto-, eine Video- oder eine CCD-Kamera, zur Aufnahme von Bildern des hinteren Augenabschnittes an die Betrachtungseinheit anschliessbar ist.

12. Vorrichtung nach einem der Ansprüche 4 bis 11, **dadurch gekennzeichnet, dass** sie einen Linsenhalter umfasst, auf welchem eine optische Einheit von Hand montiert und wieder abnehmbar ist.

13. Vorrichtung nach Anspruch 12, **dadurch gekennzeichnet, dass** der Linsenhalter zwei federnd miteinander verbundene Halteteile (32.1, 32.2) aufweist, zwischen denen die optische Einheit einklemmbar ist.

## Claims

1. Method for forming an image of a rear portion of a patient's eye (1), with a viewing unit (19) and an optical unit (12), **characterized in that** it comprises at least the following steps:
a) the optical unit is coupled in terms of movement to the viewing unit,
b) it is positioned in front of the patient's eye in such a way that it is located between the viewing unit and the patient's eye in a given relative position with respect to the patient's eye,
c) the optical unit is spatially fixed in relation to the patient's eye,
d) the optical unit is uncoupled from the viewing unit while maintaining the spatial fixing in relation to the patient's eye,
e) by moving the viewing unit, the rear portion of the patient's eye is viewed or an image of it is formed.

2. Method according to Claim 1, **characterized in that** the optical unit is positioned in front of the patient's eye in such a way that a focus of the optical unit comes to lie on a plane of the pupil of the patient's eye.

3. Method according to Claim 1 or 2, **characterized in that** the spatial fixing and the uncoupling of the lens arrangement are performed substantially at the same time.

4. Device, in particular a slit lamp unit, with a viewing unit and an optical unit for forming an image of a rear portion of a patient's eye, **characterized by** means for coupling the optical unit in terms of movement to the viewing unit, means for positioning the optical unit coupled to the viewing unit between the viewing unit and the patient's eye, means for spatially fixing the optical unit in relation to the patient's eye and also means for uncoupling the spatially fixed optical unit from the viewing unit.

5. Device according to Claim 4, **characterized in that** the viewing unit is a microscope (19), for example a Greenough microscope, and the optical unit is an auxiliary lens (12).

6. Device according to Claim 4 or 5, **characterized in that** it has a head holder (3) for the locational fixing of a patient's head and consequently of the patient's eye and the means for coupling the optical unit to the viewing unit have a lens carriage (9), which is fastened to the head holder in a horizontally movable manner, a lens holder (11) with a shaft (27) and also a guide plate (24), which is fixedly assigned locationally to the viewing unit and has a guide groove (24.1), the optical unit being fastened to the lens holder and the shaft being able to be introduced into a corresponding shaft guide (10) on the lens carriage and positioned in relation to the fixed patient's eye, and the optical unit being able to be coupled in terms of movement to the viewing unit by inserting the lower end of the shaft which has been introduced into the shaft guide on the lens carriage into the guide groove, it being possible in particular for the shaft to be inserted into the guide groove securely to prevent twisting, directly or by means of an adapter (25).

7. Device according to Claim 6, **characterized in that** the lens holder can be spatially fixed in the shaft guide on the lens carriage and/or the lens carriage can be spatially fixed on the head holder mechanically or, preferably, magnetically.

8. Device according to Claim 7, **characterized in that** the shaft guide on the lens carriage has a sleeve (35) with a ring of external teeth (36), it being possible by turning of the sleeve for a clamping mechanism for firmly clamping the shaft in the sleeve to be actuated and/or for a brake for firmly clamping the lens carriage on the head holder to be activated via the ring of external teeth.

9. Device according to one of Claims 6 to 8, **characterized in that** the lens holder, the guide plate and the adapter, if provided, are formed in such a way that the spatially fixed optical unit can be uncoupled in terms of movement from the viewing unit by horizontal and/or vertical moving out or pivoting out of the lower end of the shaft, of the guide plate or of the adapter.

10. Device according to Claims 4 to 9, **characterized in that** it is formed in such a way that the optical unit can be uncoupled from the viewing unit and spatially fixed in relation to the patient's eye substantially at the same time and without any locational displacement.

11. Device according to one of Claims 4 to 10, **characterized in that** an image acquisition device, in particular a photo camera, a video camera or a CCD camera, for recording images of the rear portion of an eye can be connected to the viewing unit.

12. Device according to one of Claims 4 to 11, **characterized in that** it includes a lens holder on which an optical unit can be mounted and removed again manually.

13. Device according to Claim 12, **characterized in that** the lens holder comprises two holding parts (32.1, 32.2) resiliently connected to each other, between which the optical unit can be clamped.

## Revendications

1. Procédé permettant la représentation d'un segment postérieur de l'oeil d'un patient (1) avec une unité d'observation (19) et une unité optique (12), **caractérisé en ce qu'**il comporte au moins les étapes suivantes :
a) l'unité optique est couplée, en ce qui concerne les mouvements, à l'unité d'observation,
b) elle est positionnée devant l'oeil du patient de manière à se trouver entre l'unité d'observation et l'oeil du patient dans une position relative donnée par rapport à l'oeil du patient,
c) l'unité optique est immobilisée dans l'espace par rapport à l'oeil du patient,
d) l'unité optique est désolidarisée de l'unité d'observation en maintenant l'immobilisation dans l'espace par rapport à l'oeil du patient,
e) le déplacement de l'unité d'observation permet d'observer respectivement de représenter le segment postérieur de l'oeil du patient.

2. Procédé selon la revendication 1, **caractérisé en ce que** l'unité optique est positionnée devant l'oeil du patient de telle façon qu'un foyer de l'unité optique s'immobilise sur un plan de la pupille de l'oeil du patient.

3. Procédé selon la revendication 1 ou 2, **caractérisé en ce que** l'immobilisation dans l'espace et la désolidarisation de la disposition de lentille ont lieu essentiellement en même temps.

4. Dispositif, en particulier lampe à fente, ayant une unité d'observation et une unité optique permettant de représenter un segment postérieur de l'oeil d'un patient, **caractérisé par** des moyens permettant le couplage, en ce qui concerne le déplacement, de l'unité optique avec l'unité d'observation, des moyens permettant le positionnement de l'unité optique couplée à l'unité d'observation entre l'unité d'observation et l'oeil du patient, des moyens permettant l'immobilisation spatiale de l'unité optique par rapport à l'oeil du patient ainsi que des moyens permettant la désolidarisation de l'unité optique immobilisée dans l'espace de l'unité d'observation.

5. Dispositif selon la revendication 4, **caractérisé en ce que** l'unité d'observation est un microscope (19), par exemple un microscope Greenough, et l'unité optique une lentille additionnelle (12).

6. Dispositif selon la revendication 4 ou 5, **caractérisé en ce qu'**il présente un support de tête (3) permettant l'immobilisation locale de la tête d'un patient et ainsi de l'oeil d'un patient, et les moyens permettant le couplage de l'unité optique à l'unité d'observation comportent un chariot porte-verre (9) fixé sur le support de tête de façon à bouger horizontalement, un porte-lentille (11) avec une tige (27), ainsi qu'une plaque de guidage (24) associée de façon localement fixe à l'unité d'observation avec une rainure de guidage (24.1), l'unité optique étant fixée sur le porte-lentille et la tige pouvant être introduite dans un guide-tige (10) correspondant sur le chariot porte-verre ainsi que positionnée par rapport à l'oeil du patient immobilisé et l'unité optique pouvant être couplée à l'unité d'observation du point de vue du mouvement par l'insertion de l'extrémité inférieure de la tige, introduite dans le guide-tige du chariot porte-verre, dans la rainure de guidage, la tige pouvant être placée dans la rainure de guidage, en particulier de façon à ne pas tourner, directement ou par l'intermédiaire d'un adaptateur (25).

7. Dispositif selon la revendication 6, **caractérisé en ce que** le porte-lentille peut être immobilisé dans l'espace, mécaniquement ou, de préférence, magnétiquement dans le guide-tige sur le chariot porte-verre et/ou le chariot porte-verre sur le support de tête.

8. Dispositif selon la revendication 7, **caractérisé en ce que** le guide-tige présente un manchon (35) avec une couronne dentée extérieure (36) sur le chariot porte-verre, la rotation du manchon permettant d'actionner un mécanisme de serrage pour le blocage de la tige dans le manchon et/ou un frein permettant le blocage du chariot porte-verre sur le support de tête pouvant être activé via la couronne dentée extérieure.

9. Dispositif selon l'une quelconque des revendications 6 à 8, **caractérisé en ce que** le porte-lentille, la plaque de guidage ainsi que l'adaptateur éventuellement présent sont configurés de manière à ce que l'unité optique immobilisée dans l'espace puisse être désolidarisée de l'unité d'observation du point de vue du mouvement par la sortie respectivement le pivotement extérieur horizontal et/ou vertical de l'extrémité inférieure de la tige, de la plaque de guidage ou de l'adaptateur.

10. Dispositif selon les revendications 4 à 9, **caractérisé en ce qu'**il est configuré de telle manière que l'unité optique puisse être essentiellement en même temps désolidarisée de l'unité d'observation et immobilisée dans l'espace sans décalage local par rapport à l'oeil du patient.

11. Dispositif selon l'une quelconque des revendications 4 à 10, **caractérisé en ce qu'**un dispositif d'imagerie, en particulier un appareil photo, une caméra vidéo ou une caméra CCD, permettant la prise d'images du segment postérieur de l'oeil, peut être raccordé à l'unité d'observation.

12. Dispositif selon l'une quelconque des revendications 4 à 11, **caractérisé en ce qu'**il comporte un porte-lentille sur lequel une unité optique peut être installée et redémontée à la main.

13. Dispositif selon la revendication 12, **caractérisé en ce que** le porte-lentille présente deux pièces de maintien (32.1, 32.2) reliées ensemble de manière élastique, entre lesquelles l'unité optique peut être serrée.
